# DEMANDE DE BREVET EUROPEEN

(11) **EP 1 095 967 A1**
(43) Date de publication de la demande: **02.05.2001**
(21) Numéro de dépôt: 99402710.0
(22) Date de dépôt: 29.10.1999
(51) Int. Cl.: C08J 7/18, C12N 5/00

(54) **Nouveaux supports de culture cellulaire porteurs de propriétés particulières et leur production**

(71) Demandeur: COMPUTER CELL CULTURE CENTER S.A., 7000 Mons (BE)
(72) Inventeur: Miller, Alain Othon André, 7000 Mons (BE); Ropson-Kenda, Nathalie Liliane Paule Ghislaine, 5080 Emines (La Bruyère) (BE)
(74) Mandataire: Desaix, Anne

(57) **Abrégé**

Cette invention se rapporte à un procédé d'obtention en continu de microsupports à deux dimensions (2D-MS) manifestant des fonctionnalités particulières pour la culture de cellules ancrage-dépendantes (CAD), comprenant une succession d'étapes telles que la production en continu de bobines de film polymère à partir de granules d'un polymère donné, l'activation dudit film polymère par tout moyen permettant de générer des groupements réactifs, le greffage covalent entre le film polymère activé et un polymère, un copolymère ou une macromolécule d'intérêt, le cas échéant un lavage pour éliminer les monomères non consommés et non fixés sur le film, suivi du séchage du film greffé et enfin la découpe du film polymère greffé par poinçonnage en continu, la taille des poinçons étant choisie en fonction de celle des microsupports 2D voulue.

## Description

La présente invention porte sur un nouveau procédé d'obtention de microsupports à deux dimensions (2D) pour la culture de cellules ancrage-dépendantes. Plus précisément, l'invention porte sur un procédé de préparation de tels microsupports adaptés à la culture en masse, en perfusion ou en clusters de cellules animales ancrage-dépendantes et manifestant des propriétés particulières telles la cryosensibilité, la biocompatibilité, la biodégradabilité ou l'adhésion spécifique. L'invention porte également sur les microsupports obtenus par ce procédé et leur utilisation. Elle porte enfin sur un dispositif qui permet de fabriquer ces microsupports en masse, de taille et de constitution homogènes.

Les cellules ancrage-dépendantes (CAD) sont dépendantes de l'adhésion à un support pour proliférer et préserver leurs fonctions cellulaires et leur viabilité. Cette dépendance constitue un goulot d'étranglement technologique pour la production de substances biologiques et pharmaceutiques secrétées par ces CAD en comparaison avec celles issues de cellules ancrage-indépendantes qui, elles, peuvent proliférer en suspension. Cette dépendance provient notamment du fait que la croissance des CAD s'arrête quand ces dernières arrivent à confluence et qu'il est nécessaire de détacher les cellules confluentes par trypsinisation. En outre, le besoin des cellules en milieu nutritif et en oxygène limite le nombre et/ou la surface des microsupports pour un volume donné de milieu de culture. Différents systèmes de culture ont été développés en vue de présenter une surface d'ancrage suffisante que pour permettre de produire des CAD à l'échelle industrielle : les "roller bottles", les systèmes "multi-tray", les fibres creuses, et les microsupports, particulièrement intéressants en terme de rapport surface/volume.

Nombre de microsupports utilisés industriellement pour cultiver en masse des cellules ancrage-dépendantes (CAD) se caractérisent par une géométrie tridimensionnelle (3D) et les plus faciles à façonner sont à géométrie sphérique. Utilisées par l'industrie biopharmaceutique pour faire de la culture cellulaire (bioréacteur jusqu'à 1000 L) en mode batch, les microbilles 3D dont un exemple est le Cytodex^{R} vendu par Pharmacia (UPPSALA, Suède) mises en suspension à 5g/l permettent d'obtenir des concentrations de l'ordre de 2.10⁶ cellules/ml, 7 % du volume de culture étant occupé par les microbilles. Celles-ci constituent une référence dans le domaine. Néanmoins, le rapport surface/volume des microsupports sphériques est peu favorable à une augmentation de la concentration en microsupports dans le bioréacteur. Si on veut cultiver des CAD à hautes concentrations (10⁷ à 5.10⁷ cellules/ml), il faut pouvoir augmenter la concentration en microsupport dans le bioréacteur. Or, il arrive rapidement un moment où le volume des microbilles gonflées représente une proportion trop importante du volume de culture, réduisant le volume de milieu disponible pour les cellules.

Une nouvelle génération de microsupports à géométrie bidimensionnelle a été développée et décrite dans EP 579 596.

Par géométrie bidimensionnelle, il faut entendre que l'épaisseur de ces microsupports tend à devenir infime et négligeable par rapport aux dimensions des cellules cultivées. Cette réduction d'épaisseur est telle qu'il n'y a aucune possibilité de croissance des cellules, ni au sein du support, ni sur sa tranche mais uniquement sur ses deux faces d'ancrage. Ces microsupports 2D (2D-MS) offrent le principal avantage d'une surface d'ancrage par unité de volume plus élevée que l'ensemble des compétiteurs 3D, telles les microbilles du type CYTODEX® mentionnées ci-dessus.

Ils permettent donc pour une occupation de volume de culture donnée dans un bioréacteur, de cultiver et d'obtenir plus de cellules par unité de volume. En effet, la surface d'ancrage externe d'une sphère (4πR²) est égale à la surface totale de deux disques (2 x 2πR²) infiniment minces situés à l'équateur et s'inscrivant dans cette sphère. Cependant, les volumes combinés de ces deux disques "équatoriaux" deviennent d'autant plus faibles que l'épaisseur du film, utilisé pour les produire, est faible, ne représentant qu'une infime fraction du volume de la sphère qui les circonscrit. Adoptant une épaisseur de 10µm pour l'ensemble des disques générés dans une sphère, la surface totale apte à l'ancrage des cellules ainsi fournie par les disques est environ 3.3 fois supérieure à la surface extérieure de la sphère, tenant compte des mouvements aléatoires de ceux-ci, en suspension.

La grande surface d'ancrage disponible pour les CAD par unité de volume des 2D-MS permet donc d'envisager la culture de cellules à hautes concentrations à l'échelle industrielle.

Une autre limitation des supports pour culture cellulaire ancrage-dépendante est celle du mode de récupération des cellules attachées à leur support tout en préservant les propriétés biologiques ou physiologiques de ces dernières. En effet, le détachement des cellules de leur support passe par un traitement enzymatique (tel la trypsine) ou un agent chélateur (tel l'EDTA), qui peut endommager non seulement les fonctions cellulaires mais également leur ré-attachement ultérieur à des supports dans le cadre de cultures en continu. Cette limitation est particulièrement préjudiciable lorsque les fonctions biologiques des cellules sont utilisées ensuite sur le plan industriel. Cette situation est fréquemment rencontrée dans le cas de production de macromolécules d'intérêt par lesdites cellules, ou encore quand l'intégralité des récepteurs ou molécules membranaires est recherchée pour leur capacité à lier des ligands ou internaliser des molécules ou des substances.

En outre, la culture à confluence des cellules ancrage-dépendantes conduit à la formation de liens intercellulaires ou de jonctions intercellulaires. Ces jonctions intercellulaires contribuent également à la fonctionnalité des cellules et leur destruction à la perte de ces fonctionnalités.

Ces limites dans la culture en masse des cellules ancrage-dépendantes conduisent également à une limite à la production industrielle de macromolécules biologiques produites par cesdites cellules qu'il s'agisse des molécules synthétisées normalement par les cellules en question ou plus généralement produites par insertion par des techniques de recombinaison génétique de gènes codant pour une protéine hétérologue. Cette limitation liée aux capacités de production de cellules ancrage-dépendantes fonctionnelles peut conduire à des prix de revient industriels des protéines que l'on souhaite exprimer et purifier, incompatibles avec un prix de vente ultérieur d'un médicament contenant ladite protéine comme principe actif.

Il existe donc un besoin réel de disposer de microsupports pour la culture de CAD présentant les spécificités suivantes :
- haut rendement de cellules par unité de volume de milieu de culture ;
- altération minimale de la viabilité des CAD consécutivement à leur décrochage des microsupports ;
- contrôle du nombre et de la spécificité des cellules susceptibles de s'ancrer sur lesdits supports, ledit contrôle permettant d'adapter ces supports, d'une part, au système de culture utilisé, d'autre part, aux fonctionnalités desdites cellules cultivées ;
- possibilité de produire à grande échelle ces microsupports pour cellules ancrage-dépendantes avec une excellente reproductibilité et à un prix de revient industriel compatible avec le prix de revient de production de cellules ou de macromolécules d'intérêt biologique.

Des tentatives pour surmonter un certain nombre d'inconvénients décrits ci-dessus, notamment la nécessité d'utiliser des enzymes ou des agents chélateurs pour décrocher les cellules de leur support, ont été décrites dans EP 387 975 et dans EP 382 214. Ces deux brevets proposent de recouvrir les supports classiques de culture cellulaire par le produit de la copolymérisation de monomères hydrophiles choisis parmi les dérivés de poly-N-alkyl-(meth)acrylamide, leurs copolymères respectifs, le poly-N-acryloyl-piperidine ou le poly-N-acryloyl-pyrrolidine dont la fonctionnalité recherchée est la cryosensibilité.

Dans cette utilisation, le polymère est choisi en fonction de la température inférieure critique ou LCST qui est une température de transition pour l'hydratation et la déshydratation du composé polymérique. Quand la LCST est inférieure à la température de culture des cellules, les cellules restent fixées sur le support du polymère pendant la phase de culture cellulaire. Elles peuvent en être décrochées consécutivement à un abaissement de la température de la culture tel que cette dernière est substantiellement inférieure à la LCST. Les méthodes d'obtention d'un polymère ou d'un copolymère avec une température LCST inférieure donnée sont décrites dans EP 382 214 B1. Tous les polymères greffés à partir des monomères cités dans cette demande de brevet sont appropriés mais non limités dans l'application à la cryosensibilité dans la présente demande. De manière générale, on peut dire que l'inclusion de monomères hydrophiles dans le processus de polymérisation tend à augmenter la LCST, alors que la présence de polymères hydrophobes tend à diminuer la LCST. Des exemples de monomères hydrophiles sont : N-vinyl-pyrrolidone, vinylpyridine, acrylamide, methacrylamide, N-methyl-acrylamide, hydroxyethyl-methacrylate, hydroxyethyl-acrylate, hydroxymethyl-methacrylate, hydroxymethyl-acrylate, acide acrylique et acide methacrylique ayant des groupes acides et leurs sels, acide vinylsulfonique, acide styrylsulfonique et N,N'-dimethylamino-ethyl-methacrylate, N,N'-diethylamino-ethyl-methacrylate, et N,N'-dimethylamino-propyl-acrylamide ayant des groupes basiques et leurs sels.

Des exemples de monomères hydrophobes sont: les dérivés acrylates et dérivés methacrylates tels que l'éthyl-acrylate, le methylmethacrylate et le glycidyl-methacrylate etc., les dérivés N-substitués-alkyl(meth)-acrylamides tels que le N-nbutyl-(meth)-acrylamide et le N-isopropyl-acrylamide etc. ainsi que le chlorure de vinyl, l'acrylonitrile, le styrene, et l'acetate de vinyl etc.

Néanmoins, les moyens décrits pour coupler ces polymères ou copolymères cryosensibles sur les supports de culture cellulaire ne permettent :
- ni le contrôle de la quantité et donc de l'épaisseur des polymères greffés, paramètre particulièrement important lorsque l'on veut contrôler la densité des cellules en culture ;
- ni d'assurer un couplage covalent du polymère ou copolymère sur le support de culture ; ceci est un inconvénient majeur dans la mesure où il ne permet pas la conservation à long terme de ces supports ni leur réutilisation.

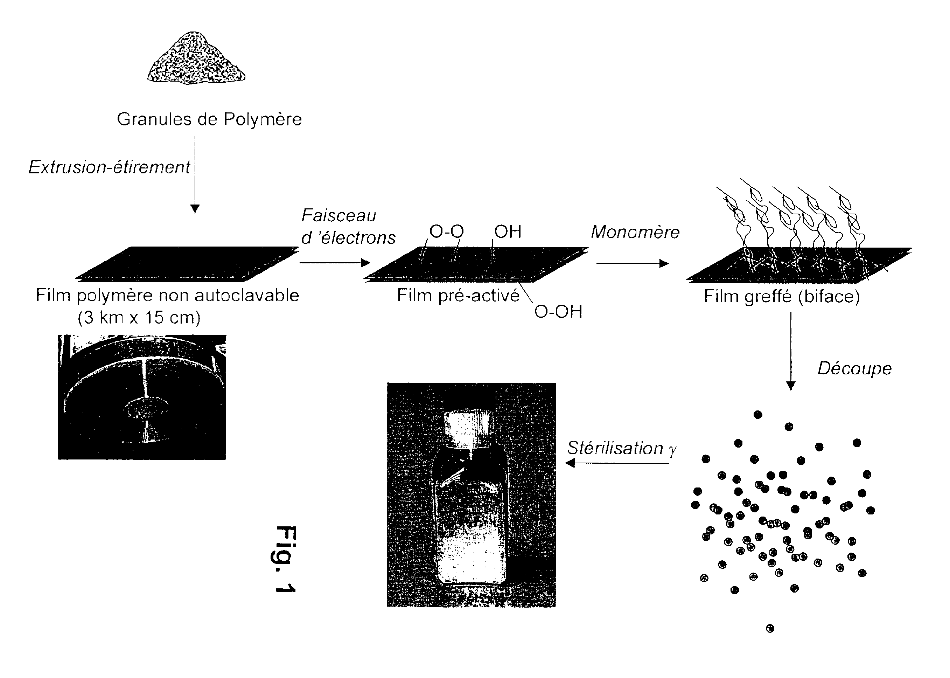

La présente invention porte sur un procédé d'obtention en continu de microsupports à deux dimensions (2D) exhibant des fonctionnalités particulières pour la culture de cellules ancrage-dépendantes (CAD). Il est illustré dans la figure 1 et comprend au moins la succession d'étapes suivantes :
- une production en continu de bobines de film polymère d'une épaisseur inférieure ou égale à 35 µ à partir de granules d'un polymère donné ;
- une activation dudit film polymère par tout moyen permettant de générer des groupements réactifs notamment des radicaux ou des fonctions peroxydes, hydroperoxydes ou amines ;
- un greffage covalent entre le film polymère activé et un polymère, un copolymère ou une macromolécule d'intérêt dont la fonctionnalité est recherchée, ledit greffage étant obtenu par immersion du film dans une solution de monomère, la copolymérisation étant amorcée par les radicaux libres créés par l'activation sous rayonnement β, le temps d'immersion étant directement corrélé à l'épaisseur recherchée du polymère greffé sur le film ;
- le cas échéant un lavage pour éliminer les monomères non consommés et non fixés sur le film ;
- une découpe du film polymère par un procédé choisi en fonction de la géométrie et de la taille des microsupports 2D recherchées ;
- le cas échéant, une étape de stérilisation, soit par autoclavage, soit par rayonnement, la méthode de stérilisation étant bien entendu choisie en fonction du matériau à stériliser. Dans le cas où le matériel n'est pas autoclavable, il doit être stérilisé par une méthode physique (rayonnement γ ou β) ou chimique (mélange isopropanol/H₂O 70/30 % (v/v)).

Un procédé particulièrement avantageux comprend une découpe par poinçonnage en continu, la taille des poinçons étant celle de la taille des microsupports 2D recherchée.

C'est la combinaison de ces différentes étapes incluant une activation suivie d'un greffage covalent d'un film polymère (substrat) préalablement mis en oeuvre puis une découpe dudit film substrat porteur du polymère, copolymère ou macromolécule greffés de façon covalente par un procédé permettant l'obtention de microsupports 2D homogènes en taille et géométrie qui fait toute l'originalité de l'invention.

En ce qui concerne la matière première mise en oeuvre sous forme de film, la nature du polymère ne peut être quelconque. Il est souhaitable suivant l'invention que le matériau soit :
- de densité comprise entre 0.9 et 1.25g/cm³ et de préférence entre 1 et 1.1 g/cm³ pour permettre une culture agitée en suspension dans du milieu de culture (en bioréacteur), sans risque de sédimentation ou de flottation.
- transparent pour permettre une analyse aisée des cellules au cours de leur croissance, en continu dans le bioréacteur. Par transparent, on entend que dans des longueurs d'ondes comprises entre 400 nm et 1000 nm, la lumière traverse les microporteurs sans atténuation importante de l'intensité du faisceau lumineux émergeant par rapport à l'intensité du faisceau de lumière incident. Une adsorption inférieure à 1 % est considérée comme tout à fait avantageuse ;
- caractérisé par une balance hydrophobe/hydrophile adéquate : s'il est plutôt hydrophobe, il doit avoir un angle de contact tel qu'il soit suffisamment mouillable en milieu aqueux et s'il est plutôt hydrophile, il ne doit pas gonfler dans l'eau ; autrement dit, l'angle de contact θ entre la surface du matériau et une goutte de milieu aqueux doit être compris entre 30° et 90°, l'angle de contact étant défini comme l'angle formé entre la surface du matériau et la tangente de la goutte au point triple d'intersection entre la goutte, la surface du matériau et l'air.

Un angle θ = 0 correspond à un mouillage parfait et la surface de liquide est parallèle à la surface du matériau. Un angle θ > 90 % correspond à une situation d'absence de mouillage et des gouttes restent formées en surface du matériau. Un angle de contact θ compris entre 30 et 90 ° correspond à un mouillage imparfait correspondant à un étalement partiel de la goutte sur le matériau.

Suivant l'invention, le matériau polymère pouvant être utilisé pour produire les films (substrat) peut ainsi être le polystyrène, le polyéthylène, le polyéthylènetéréphtalate ou le polycarbonate ou tout copolymère incluant majoritairement ces matériaux polymères plutôt hydrophobes. En tant que film plus hydrophile, le matériau utilisé peut également être en cellophane ou en polyester aliphatique de type polylactide ou polyhydroxybutyrate et tout copolymère incluant majoritairement ces matériaux plus hydrophiles.

Lorsque la nature du polymère mis en oeuvre est de type polyester aliphatique, le film est par essence biorésorbable/biodégradable et, dans ce cas, il peut être utilisé en tant qu'implant dans l'organisme vivant si le polymère mis en oeuvre est de grade biomédical de préférence reconnu par la FDA.

De préférence, l'épaisseur du film utilisé pour produire les microsupports est entre 10 et 25µ.

La production en continu de bobines de film polymère mince ou ultra-mince est réalisée par la technique "d'extrusion-étirement" à partir de granules d'un polymère donné. Cette matière première est chauffée pour atteindre l'état fondu, puis extrudée au travers d'une vis tournante et moulée entre deux plaques pour produire un film polymère épais. A la sortie de l'extrudeuse, le film assez épais est alors étiré à chaud, soit dans un seul sens, soit dans deux sens orthogonaux l'un par rapport à l'autre pour produire alors un film non rétractable dont l'épaisseur est beaucoup plus faible et contrôlable à façon (entre 10 et 35µ) sur toute la largeur de la bobine produite, ceci dans les limites des propriétés thermiques et mécaniques de la matière première.

La technique d"'extrusion-soufflage" est une alternative de mise en oeuvre de film en continu. La variante se situe au niveau de la seconde étape, à savoir, l'injection d'air entre deux parois de films qui a pour rôle d'étendre la matière et par conséquent d'obtenir une épaisseur de film plus faible. D'autres techniques de mise en oeuvre de films minces sont décrites dans d'autres applications de la chimie des polymères (tels que les biosensors): le "spin-coating" ou le "solvent-casting" mais ces deux techniques sont plus souvent utilisées pour faire des feuilles ou des disques de petites tailles et sont moins appropriées pour produire des bobines de film en continu.

Dans une deuxième étape, le film polymère, qui peut avantageusement se présenter sous une forme enroulable d'une largeur comprise entre 5 cm et 60 cm et une longueur d'au moins 3 km, subit ensuite une activation afin de permettre de générer des groupements réactifs, susceptibles de former des liaisons covalentes avec d'autres groupements réactifs de la substance que l'on souhaite greffer. Dans ce sens, par substance, on entend des monomères ou polymères organiques ayant des propriétés particulières, notamment des propriétés de cryosensibilité ou de biocompatibilité; il peut s'agir également de macromolécules biologiques qui ont une affinité spécifique pour certains récepteurs cellulaires : les microsupports 2D résultant d'un tel greffage permettent alors l'adhésion sélective de certains types de cellules présentes dans un échantillon cellulaire initial comprenant un mélange de cellules. A titre d'exemple, on peut citer des cellules de la peau (kératinocytes) parvenues à différents stades de différenciation, ou encore des cellules résultant de l'insertion, l'activation ou la répression d'une fonction particulière, notamment par l'insertion d'un gène porteur de ladite fonction ou porteur d'une fonction régulatrice de l'expression d'un gène cellulaire.

Quatre procédés sont employés pour modifier la structure chimique des polymères et générer des groupements réactifs. Il s'agit des faisceaux d'électrons et, plus particulièrement, de rayonnements β, des décharges couronnes ou corona, du traitement UV et, enfin, des plasmas. Pour chaque procédé, deux paramètres vont orienter le choix de la méthode selon les propriétés recherchées pour le matériau soumis à ces rayonnements :
- la nature des groupes chimiques induits dans le polymère par l'activation,
- la profondeur de traitement dans l'épaisseur du matériau.

Dans tous les cas, l'activation consiste à soumettre le support à un rayonnement électromagnétique qui engendre une cassure des liaisons et la création de radicaux libres, de fonctions peroxydes, hydroperoxydes ou amines.

Si besoin est, et selon des méthodes déjà décrites, des molécules espaceurs peuvent être, le cas échéant, greffées via les radicaux libres ainsi générés. Leur fonction est d'augmenter la longueur du lien entre les sites réactifs et les monomères, polymères ou macromolécules que l'on souhaite lier de façon covalente au film polymère, et par conséquent d'augmenter la mobilité de ceux-ci.

L'activation consiste à soumettre ledit film à un bombardement électronique. Ce bombardement est réalisé, de préférence, sous atmosphère inerte. Dans le procédé de l'invention, il est essentiel que l'étape d'activation précède l'étape de greffage. En effet, lorsque ces deux étapes sont simultanées comme dans le cas du brevet EP 382 214, le monomère que l'on souhaite greffer est alors soumis lui aussi au rayonnement et il se crée une quantité très importante d'homopolymères libres en solution qui peuvent s'adsorber à la surface du support et qu'il faut ensuite éliminer au lavage. Dans ce cas, il est alors difficile de s'assurer que toutes les chaînes libres non greffées sont éliminées par le lavage, et que ces chaînes libres adsorbées ne se dissolvent pas dans le milieu de culture lors du détachement cellulaire par contraste thermique.

Les conditions d'activation sont choisies en fonction d'un certain nombre de paramètres parmi lesquels figurent au moins :
- la nature du film polymère à greffer ;
- la nature du copolymère ou des macromolécules que l'on souhaite coupler de façon covalente au film polymère ;
- le caractère discontinu ou continu du procédé d'activation : le support greffé qui sera ensuite découpé peut en effet être traité de façon statique ou en continu par défilement du film.

Quand le procédé d'activation est continu, la vitesse de défilement du film peut varier de 0,1 à 10 m par minute et être établie en fonction de la dose totale d'irradiation requise pour activer le film et de la puissance de l'irradiateur fixée selon la résistance thermique du film.

Dans le cas de l'irradiation de film polystyrène par rayonnement β ou γ, on peut augmenter la puissance de l'irradiateur jusqu'à 6 milliampères (mA) ; au-delà, il y a surchauffe et déformation du film. Si on diminue sensiblement la vitesse de défilement du film, avec une intensité fixée à 6 mA, on peut atteindre des doses de 80 à 200 Kgray au maximum en un seul passage sous l'irradiateur.

Le Kilogray ou joule par gramme est une unité représentative de la dose et dépend des caractéristiques de l'unité de faisceau d'électrons.

Lorsqu'une décharge couronne (ou corona) est utilisée, elle est émise sous une tension de plusieurs milliers de volts et à des fréquences la situant dans le domaine des kHz. Ce procédé s'utilise sous atmosphère ambiante. L'amplitude géométrique de l'arc corona est de quelques millimètres pour les systèmes les plus classiques à quelques centimètres pour les systèmes à arc soufflé. La décharge est réalisée avec des électrodes parallèles se situant de part et d'autre de l'objet. L'utilisation des décharges couronnes pour l'activation du film a l'avantage d'être un traitement sous atmosphère ambiante.

L'activation du film polymère par plasma froid est réalisée également à l'aide d'électrodes qui émettent des décharges dans le domaine des radiofréquences.

Un plasma est obtenu par ionisation à l'aide d'une source haute fréquence d'un gaz ou d'un mélange de gaz introduit dans une enceinte mise sous une pression résiduelle de quelques millibars. Ce procédé coûteux est également difficile à mettre en oeuvre en continu sur un film polymère. Néanmoins ces trois types d'activation : faisceau d'électrons, β ou γ, décharges couronnes ou plasma sont appropriés pour générer des groupements réactifs.

Suivant l'invention, la technique de radiogreffage de monomères de type acrylamide ou vinylique tels que décrits plus haut à la surface d'un mince film polymérique de type polystyrène ou polyesther aliphatique doit donc être initiée par rayonnement quelle que soit la nature de celui-ci. L'étape de greffage est réalisée ensuite directement par immersion en plongeant le film polymère pré-activé dans une solution du monomère, d'un mélange de plusieurs monomères ou de macromolécules sélectionnés.

S'il s'agit d'un monomère organique, sa copolymérisation à la surface du film polymère est amorcée par les radicaux libres créés durant la pré-irradiation, et les chaînes polymères formées sont liées par covalence au film. La réaction est instantanée mais, suivant la nature du film, elle pourra se prolonger pendant quelques secondes, voire quelques minutes pour arriver à un taux de greffage maximum. Ce taux de greffage est directement lié à la dose d'irradiation, à la concentration en monomères dans le bain d'imprégnation, et au temps de la réaction.

Le procédé selon l'invention comprend ainsi entre autres l'optimisation des quatre paramètres cités ci-dessus, à savoir : nature et dose d'irradiation, durée du greffage, concentration des monomères et nature du solvant dans le bain de greffage, afin d'obtenir une couche de polymère, copolymère ou de macromolécules spécifiques greffés de façon covalente et d'épaisseur souhaitée. De fait, comme le montre l'exemple 2 ci-après, l'épaisseur de la couche de polymère, de copolymère organique, ou de macromolécules spécifiques déposée est déterminée par analyse « X-ray photon spectroscopy » (XPS) et dépend directement de la durée de l'étape de greffage par imprégnation dans le bain.

Dans le cas où l'étape de pré-irradiation est réalisée sous air, les groupements réactifs créés sont susceptibles d'être oxydés instantanément en présence de l'air. Dans ce cas, l'étape de greffage est réalisée extemporanément par l'immersion dans un bain contenant un composé ad hoc qui permet de régénérer les radicaux réactifs.

Dans le procédé de l'invention, l'étape de greffage peut être le cas échéant suivie d'une étape de lavage qui consiste à éliminer les résidus du monomère, du polymère ou des macromolécules biologiques qui n'ont pas été consommés et/ou polymérisés à la surface du film polymère. Ces rinçages se réalisent généralement dans un mélange d'isopropanol dans l'eau (70/30 % v/v) jusqu'à ce qu'il n'y ait plus aucune trace de réactifs et/ou de produits dans les phases de lavage. Dans l'application à la culture de cellules, il est essentiel que le lavage soit le plus efficace possible du fait de la cytotoxicité des monomères ou polymères d'acrylamide et dérivés.

Dans le procédé de l'invention, différentes natures de polymères, copolymères ou macromolécules peuvent ainsi constituer une couche comprise entre 1 et 10 nanomètres sur laquelle les cellules adhérentes se fixent et prolifèrent. Lorsque la cryosensibilité totale ou partielle est recherchée, le polymère ou le copolymère d'intérêt est choisi parmi les dérivés de poly-N-alkyl (meth)acrylamides, leurs copolymères respectifs, le poly-N-acryloyl-piperidine ou le poly-N-acryloyl-pyrrolidine. Lorsque la biocompatibilité est recherchée, le traitement de surface implique par exemple le greffage covalent de polymère hydrophile de type polyoxyde d'éthylène PEO aminé à la surface d'un film polymère pré-exposé à un plasma d'allylamine pour générer des fonctions amines en surface, et ce par l'intermédiaire d'un agent de couplage chimique adéquat, comme par exemple le chlorure de cyanure. Ce type de traitement est décrit dans (J. Biomed. Mater. Res. 1991, 25, 1547).

Quand la propriété recherchée est une adhésion sélective de cellules animales, les méthodes classiques de greffage de macromolécules sur les supports telles que décrites notamment dans les techniques de chromatographie par affinité utilisant des anticorps, des aptamères ou des molécules obtenues par chimie combinatoire, peuvent être utilisées. L'homme du métier pourra se reporter, pour un descriptif détaillé de ces techniques, à (J. Cell. Biol. 1991, 114, 1089 § 1990, 110, 777, J. Biol. Chem. 1992, 267, 14019 § 10133, Artif. Organs 1992, 16, 526, Macromolécules, 1993, 26, 1483). Les macromolécules biologiques (oligopeptides, oligonucléotides etc.), susceptibles d'être greffées avantageusement sur les supports préparés par un procédé selon l'invention, sont des ligands spécifiques de récepteurs cellulaires permettant de réaliser la croissance d'un certain type de cellules dans un milieu de culture au détriment d'autres types cellulaires qui pourraient y être mélangés. De manière encore plus particulière, cela pourrait permettre de multiplier les cellules qui expriment, soit de façon naturelle, soit résultant d'une recombinaison génétique in vitro de ces cellules exprimant une macromolécule spécifique au niveau de leur membrane.

Dans le procédé de l'invention, le film polymère substrat sur lequel ont été greffés un polymère, un copolymère ou une macromolécule d'intérêt, est ensuite découpé par un procédé choisi en fonction de la géométrie et de la taille des microsupports 2D voulues.

Une mise en oeuvre préférée dans la présente invention est le poinçonnage, la taille des poinçons étant celle des microsupports 2D produits.

Suivant une forme de réalisation préférée de l'invention, les microsupports ont une épaisseur de préférence inférieure ou égale à 25 µ ainsi qu'une forme de disque. La dernière étape du procédé de production de tels 2D-MS greffés comprend donc un découpage du film greffé polymère (substrat) mince ou ultra-mince en particules micrométriques caractérisées par une géométrie bidimensionnelle et comportant deux faces d'ancrage sur chacune desquelles les cellules s'accrochent et prolifèrent sans pénétration possible des cellules entre les deux faces.

Dans notre procédé, nous avons sélectionné une technique de découpe par poinçonnage qui permet d'accéder à une excellente qualité de microdisques produits, en terme d'homogénéité de taille, de forme et d'épaisseur des microdisques fournis, et en terme d'absence de débris.

L'homogénéité de taille (homodispersité) est essentielle à la synchronie des différentes étapes de la croissance des cellules. En effet, la présence de microsupports de diverses tailles impliquerait que ceux de petite taille atteindraient la confluence avant ceux de grande taille. Dans ce cas, les CAD qui ont atteint la confluence plus tôt, peuvent en partie se décrocher, mourir et relarguer de l'ammoniac, de l'acide lactique et d'autres toxines qui sont délétères pour la croissance des CAD proliférant sur les microsupports de plus grande taille.

Cette étape est réalisée par poinçonnage du film polymère uniquement préactivé ou préactivé puis greffé par des poinçons circulaires de diamètre choisi (par exemple 150 µ) en carbure céramique. Le film polymère est poinçonné à une fréquence de découpe (battements par minute) optimisée en fonction de la vitesse d'avancement du film polymère greffé.

D'autres techniques de découpe peuvent être envisagées. D'une part, les systèmes d'embossing utilisant un couteau rotatif constitué de gravures enfichées sur un cylindre d'impression de diamètre donné, un second cylindre de même diamètre servant de presse ou "d'empreinte". Il y a un nombre de lignes de poinçons donnés sur les 360° du cylindre (définis en fonction de l'espacement entre poinçons et du diamètre du cylindre) et un nombre de poinçons par ligne donné (défini en fonction de la longueur du cylindre). La vitesse de découpe d'une bobine de film passant entre les deux cylindres et donc la productivité doit être différente de notre procédé. C'est une technique fort utilisée par les sociétés qui commercialisent des étiquettes de toutes tailles et formes. Dans ce cas, la pression qu'il faudrait appliquer pour la découpe risque de limiter le procédé.

L'invention porte également sur les microsupports à deux dimensions (2D) doués de fonctionnalités particulières pour la culture de cellules ancrage dépendantes (CAD), tels qu'obtenus par un procédé décrit ci-dessus, caractérisés en ce que l'épaisseur du film polymère est comprise entre 10 et 35 µ, et celle du polymère, du copolymère ou d'une macromolécule d'intérêt greffés de façon covalente ont une épaisseur comprise entre 1 et 10 nm.

La succession des étapes de la fabrication de 2D-MS greffés est donnée à la figure 1.

Lorsque les 2D-MS sont cryosensibles, une épaisseur minimale de 5 nanomètres pour la couche greffée est nécessaire si l'on souhaite que les cellules à confluence soient détachées de manière quantitative de leur support. Des épaisseurs plus faibles sont recherchées lorsque l'on souhaite détacher partiellement et non quantitativement par contraste thermique des CAD à confluence.

L'invention porte également sur un dispositif de préparation en continu de support 2D-MS doués de propriétés particulières, préparé selon un procédé tel que décrit ci-dessus et mettant en oeuvre une fixation covalente d'un polymère, copolymère ou macromolécules biologiques sur un substrat constitué d'un film polymère, ledit dispositif comprenant :
- un système de déroulement/enroulement du film polymère, l'entraînement du film étant effectué à une vitesse choisie au niveau de chacune des étapes : activation, greffage, poinçonnage ;
- un irradiateur permettant d'activer la surface du film ;
- un bain de greffage destiné à contenir la solution de monomères, polymères ou macromolécules à greffer, dans lequel le film polymère préactivé passe, de façon continue, à une vitesse déterminée, entraîné par le système d'enroulement/déroulement ;
- un outil de découpe permettant de réaliser le poinçonnage du film lors de son déroulement.

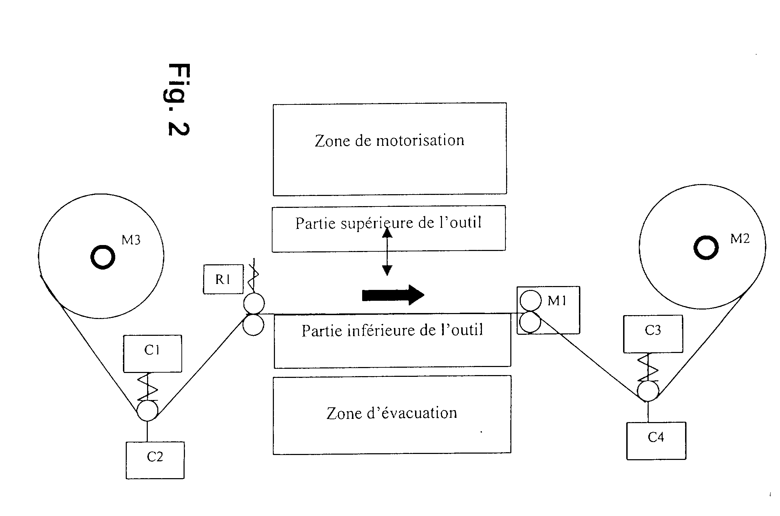

La figure 2 représente le schéma du dispositif de l'étape de découpe en continu du film (sous forme de bobines). Le système enrouleur-dérouleur sert d'approvisionnement et d'évacuation à un outil de découpe. De plus, cet outil fonctionnera 24 heures sur 24. M1 est le moteur régissant l'avance de bande, M2 est le moteur d'enroulement de la bobine et M3 est le moteur de déroulement de la bobine. C1 représente le capteur de mise en marche de M3, C2 est le capteur d'arrêt de M3, C3 représente le capteur de mise en marche de M2 et C4 est le capteur d'arrêt de M2. Enfin, R1 indique le système de freinage de bande.

De façon préférée, le système de déroulement/enroulement du film permet l'avancement dudit film à une vitesse comprise entre 0,1 et 8 m par minute. La vitesse de déroulement/enroulement est beaucoup plus lente au stade de la découpe que lors des étapes d'activation et de greffage.

Le coeur du dispositif est constitué d'un bloc outil sur lequel sont enfichées, dans le sens de la longueur du film, des rangées de poinçons circulaires et le bloc empreinte correspondant. A titre d'exemple, pour un film de 5 cm de largeur, le bloc-outil comprendra 9 rangées de 50 poinçons chacune. Cette configuration du bloc-outil en ligne dans le sens de la longueur plutôt que de la largeur augmente la sécurité de maintenance. Sur une même ligne de poinçons, l'espacement entre ceux-ci est de 0,25 mm ; le pas régulier entre 2 lignes est de 0,20 mm. Il est clair que la disposition citée ci-dessus, le diamètre des poinçons et leur espacement est une proposition optimale mais peut bien entendu être adaptée en fonction des besoins et n'a aucun caractère contraignant.

Au fur et à mesure du poinçonnage, les microsupports greffés 2D-MS sont ensuite recueillis en continu. A titre d'exemple, à partir d'une bobine de polystyrène greffée d'une largeur de 5 cm et d'une longueur de 3 km, le dispositif selon l'invention comprenant des poinçons de diamètre 150 µ permet d'obtenir environ 1 kg de confettis (microsupports 2D-MS) de 150 µ de diamètre. Sachant que la densité surfacique de la matière est de 26,25 g/m², le nombre minimum de particules obtenues égale 2,38 10⁹ microsupports par bobine ou par kilo de microsupports produits. Dans la configuration citée ci-dessus à titre d'exemple, le rendement de découpe est de 28 %.

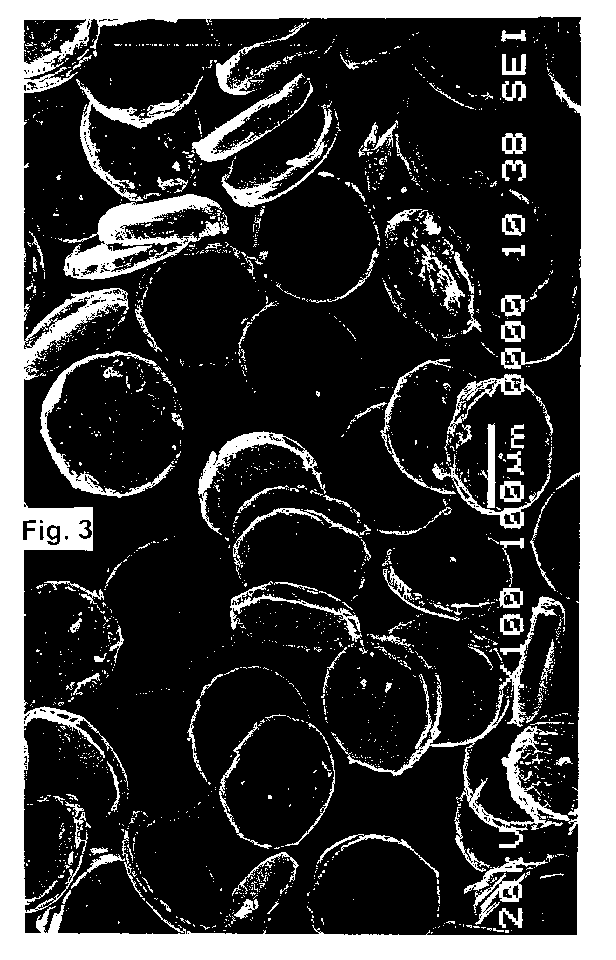

La figure 3 représente les microsupports 2D-MS ainsi obtenus.

Les microdisques peuvent être récupérés dans des réceptacles positionnés directement sous la matrice de poinçonnage.

Les exemples ci-après permettent, sans les limiter, de montrer les avantages du procédé et des supports pour culture cellulaire tels qu'obtenus par ce procédé.

### Exemple 1 - Radiogreffage via la technique de pré-irradiation par faisceau d'électrons sous azote :

Dans un premier temps, nous avons recherché à optimiser et à définir les paramètres de radio-greffage en discontinu (dose totale d'activation, nature du solvant, concentration du monomère, température du bain de greffage, temps de greffage) sur des feuilles (film de 5 x 10 cm² en polystyrène de 25 µ d'épaisseur) avec un accélérateur d'électrons statiques de recherche de type Van der Graaf, caractérisé par une faible puissance et un faible débit de doses comparativement aux appareils industriels. Ces feuilles sont placées préalablement dans des flacons de culture de 75 cm² pré-dégazés et irradiés à sec sous atmosphère inerte (azote) de la façon suivante :

Les flacons de culture en polystyrène contenant le film de polystyrène sont lavés deux fois dans un mélange Isopropanol / H₂O 70/30 % (v/v) et séchés sous flux d'azote pendant 15 minutes.

Les flacons préalablement dégazés sont placés sous un irradiateur EB statique haute énergie (10 Mev). Un accélérateur d'électrons (10 MeV) de type Van der Graaf dont l'intensité est fixée à 1 mA et le débit de dose fixé à 10 Kgray/min (1 Mrad/min), est utilisé. Les flacons sont dès lors irradiés sous le faisceau pendant un temps fixé (en minutes) afin d'absorber une dose totale donnée (en Kgray ou Joule/g). La dose déposée est calibrée préalablement avec un dosimètre.

A la sortie de l'irradiateur, les flacons sont immédiatement mis en contact avec la solution (H₂O), stock de monomère (concentration en poids de 10 à 40 %) sous atmosphère d'azote. La solution stock de monomère, fraîchement préparée, est transférée dans le flacon en polystyrène pré-irradié par un système de poussée d'azote. La solution de greffage, une fois transférée dans le flacon pré-irradié, est équilibrée à une température donnée (de 25 °C à 60 °C). Le temps de greffage (de 0.5 à 24 heures) est varié à une température donnée afin de vérifier l'influence de divers paramètres sur l'épaisseur de la couche de Poly-N-isopropylacrylamide à la surface du film comme du flacon de culture en polystyrène. Les solutions de greffage sont éliminées des flacons après un temps donné puis les flacons ainsi que les films greffés sont lavés trois fois puis séchés.

### Exemple 2 - greffage de N-lsoPropylAcrylAmide (NIPAAm) sur un film de polystyrène :

2.1 Etude de l'épaisseur du dépôt en fonction du temps de réaction :
Des films de 25 microns d'épaisseur sont irradiés sous azote en utilisant un accélérateur d'électrons de type Van der Graaf (10 Mev) et une dose totale de 250 Kgray. Les films irradiés sont immédiatement immergés dans une solution aqueuse de NIPAAm (10 % en poids) qui a été dégazée préalablement pendant 30 min avec de l'azote. La température de la solution de greffage est de 60 ° C. Différentes surfaces greffées ont été obtenues en variant la concentration de monomère dans la solution de greffage et le temps de réaction. Quand les réactions de greffage sont terminées, les films greffés sont lavés, séchés et analysés par "X-ray Photon Spectroscopy" (XPS), afin de déterminer la composition chimique d'extrême surface.
L'épaisseur de dépôt en fonction du temps de réaction est présentée dans le tableau 1 ci-dessous.

**TABLEAU 1 :**

| Films greffés selon l'exemple 1 - Analyse XPS | | | | | |
|---|---|---|---|---|---|
| Echantillons de films (Temps de greffage) | % Atomiques Expériment. | | | Epaisseur de dépôt (en A) | % décrochage cellulaire (par comptage) |
| | C | O | N | | |
| Polystyrène natif | 98,5 % | 1,5 % | 0 % | - | - |
| Polystyrène greffé (1/2 h) | 92,3 % | 4,1 % | 3,6 % | 38 Å | 65,5 % |
| Polystyrène greffé (1 h) | 78,7 % | 11,3 % | 10 % | 47 Å | 79,0 % |
| Polystyrène greffé (3 h) | 75,7 % | 13,1 % | 11,2 % | >50-60 Å | 96,5 % |
| Poly-N-IsoPropyl-AcrylAmide natif | 75,0 % | 12,7 % | 12,3 % | - | - |

Le pic C_{1S} du carbone du polystyrène natif peut être décomposé en deux composantes (à 284.8 eV et à 291.5 eV) qui correspondent respectivement à du carbone impliqué dans des liaisons hydrocarbures et au pic « shake-up » caractéristique des composés aromatiques. Selon la littérature, ce dernier pic a une intensité de 7 % par rapport au carbone total pour le polystyrène.
L'épaisseur du film a été calculée par le rapport de surface (%) du pic C_{1S} « shake up » caractéristique du polystyrène et du pic C_{1S} total pour l'échantillon de film greffé analysé à un angle de collecte des électrons de 90 ° (Analyse quantitative). Dans le cas où le pic C_{1S} « Shake up » caractéristique du polystyrène pur, n'est plus détecté en XPS, l'épaisseur du dépôt greffé est supérieure à la profondeur d'analyse de la technique XPS, à savoir 50 à 60 Å.
2.2 Des films de 25 µ d'épaisseur sont irradiés en discontinu sous azote en utilisant un accélérateur d'électrons de type Van der Graaf (10 Mev) et une dose totale de 150 Kgray. Les films irradiés sont immédiatement immergés dans une solution d'isopropanol de NIPAAm (10 % en poids) qui a été dégazée préalablement pendant 30 minutes avec de l'azote, pendant 3 heures. La température de la solution de greffage est de 20 °C. Aucun dépôt de polyN-IPAAm n'est observé.
2.3 Des films de 25 µ d'épaisseur sont irradiés, en discontinu, sous air en utilisant un accélérateur d'électrons de type Electrocurtain opérant à 150 KeV et une dose totale de 100 Kgray. Les films irradiés sont immédiatement piégés dans de l'azote liquide (pendant le transport) et conservés à très basse température (congélateur à -180°C) pour préserver la stabilité des fonctions peroxides/hydroperoxides générées en surface. Après décongélation, les films pré-irradiés sont immédiatement immergés dans une solution aqueuse de NIPAAm (10 % en poids) contenant du chlorure ferreux (0,1 % en poids) qui a été dégazée préalablement pendant 30 minutes avec de l'azote. Cet agent réducteur permet de décomposer chimiquement les fonctions oxydées en surface du film en sites initiant la polymérisation radicalaire du N-IPAAm. La température de la solution de greffage est 37°C. Le rapport molaire monomère/agent réducteur et le temps de réaction ont un effet sur le rendement de greffage et l'épaisseur de dépôt.
2.4 Des films de 25 µ d'épaisseur dont une des deux faces a été masquée par une feuille d'aluminium, sont irradiés sous azote et utilisant un accélérateur d'électrons de type Van der Graaf (10 Mev) et une dose totale de 250 Kgray. Les films irradiés sont immédiatement immergés dans une solution aqueuse de NIPAAm (10 % en poids) qui a été dégazée préalablement pendant 30 minutes avec de l'azote. La température de la solution de greffage est de 60 °C. Des supports greffés sélectivement sur une des deux faces ont été obtenus.

Nous pouvons conclure de ces différents essais que les rendements de greffage les plus importants, menant à une épaisseur de dépôt de plus de 5 nanomètres, ont été obtenus lors de l'étape de pré-irradiation, avec une dose totale d'irradiation de 250 Kgray (temps d'irradiation 25 min), en laissant les films préactivés en contact avec le monomère durant au moins 2 heures. Lorsque le temps de greffage est plus court, les rendements de greffage, et par conséquent des épaisseurs de dépôt plus faibles (inférieures à 5 nanomètres) ont été obtenues à la surface du film polystyrène. De telles surfaces ne confèrent que partiellement les propriétés et fonctionnalités recherchées.

## Revendications

1. Procédé d'obtention en continu de supports à deux dimensions (2D) doués de fonctionnalités particulières pour la culture de cellules ancrage-dépendantes (CAD), comprenant au moins la succession d'étapes suivantes :
- une production en continu de bobines de film polymère d'une épaisseur inférieure ou égale à 35 µ et d'une densité comprise entre 0.9 et 1.25 g/cm³ à partir de granules d'un polymère donné,
- une activation dudit film polymère par tout moyen permettant de générer des groupements réactifs notamment des radicaux et/ou fonctions peroxydes, hydroperoxydes, ou amines ;
- un greffage covalent entre le film polymère activé et un polymère, un copolymère ou une macromolécule d'intérêt dont la fonctionnalité est recherchée, ledit greffage étant obtenu par immersion du film dans une solution de monomère, la copolymérisation étant amorcée par les radicaux libres crées par l'activation sous rayonnement β, le temps d'immersion étant directement corrélé à l'épaisseur recherchée du polymère greffé sur le film ;
- le cas échéant un lavage pour éliminer les monomères non consommés et non fixés sur le film, suivi du séchage du film greffé,
- une découpe du film polymère greffé par poinçonnage en continu, la taille des poinçons étant choisie en fonction de celle des supports 2D voulue.

2. Procédé selon la revendication 1 dans lequel le film polymère a un angle de contact θ compris entre 30 ° et 90 °.

3. Procédé selon la revendication 1 dans lequel l'activation est réalisée par décharge Corona, plasma ou bombardement d'électrons.

4. Procédé selon la revendication 3 dans lequel l'activation électronique du film polymère est réalisée par bombardement avec un rayonnement β, sous atmosphère inerte.

5. Procédé selon les revendications 1 à 4 dans lequel l'épaisseur de la couche polymère greffée à la surface du film est déterminée par la combinaison des paramètres suivants : la dose totale d'irradiation β reçue par le film, la concentration en monomère dans le bain de greffage, le temps de greffage, la température de greffage.

6. Procédé selon l'une des revendications précédentes dans lequel le polymère ou le copolymère d'intérêt est choisi parmi les dérivés de poly-N-alkyl (meth)acrylamides, leur copolymères respectifs, le poly-N-acryloyl piperidine ou le poly-N-acryloyl pyrrolidine dont la fonctionnalité recherchée est la cryosensibilité.

7. Procédé selon les revendications 1 à 5 dans lequel le polymère ou le copolymère d'intérêt est un polymère hydrophile de type polyoxyde d'ethylène PEO aminé dont la fonctionnalité recherchée est la biocompatibilité.

8. Procédé selon les revendications 1 à 5 dans lequel la ou les macromolécule(s) d'intérêt sont un ou des ligands spécifiques de récepteurs cellulaires et dont la fonctionnalité recherchée est l'adhésion sélective des cellules porteuses de ce(s) récepteur(s).

9. Procédé selon la revendication 6 dans lequel quand le film polymère (substrat) est en polystyrène et quand le polymère greffé est choisi parmi les dérivés de poly-N-alkyl (meth)acrylamides, leur copolymères respectifs, le poly-N-acryloyl-piperidine ou le poly-N-acryloyl-pyrrolidine, une épaisseur de 40 à 60 Å est obtenue par la combinaison d'une dose totale d'irradiation de 50 à 250 KGray, d'un temps et d'une température d'immersion dans la solution de monomères, respectivement de 1 à 3 heures et de 50 à 70 degrés.

10. Procédé selon la revendication 1 dans lequel l'activation est antérieure au greffage du polymère ou du copolymère d'intérêt dont la fonctionnalité est recherchée.

11. Microsupports à deux dimensions (2D) doués de fonctionnalités particulières pour la culture en masse de cellules ancrage-dépendantes (CAD), tels qu'obtenus par un procédé selon l'une quelconque des revendications précédentes, caractérisés en ce que l'épaisseur du film polymère est comprise entre 10 et 35 microns, et celle du polymère, du copolymère ou d'une macromolécule d'intérêt greffés de façon covalente ont une épaisseur comprise entre 1 et 10 nm.

12. Microsupports selon la revendication 11 dans lesquels le polymère greffé est un polymère cryosensible choisi parmi les dérivés de poly-N-alkyl (meth)acrylamides, leurs copolymères respectifs, le poly-N-acryloyl-piperidine ou le poly-N-acryloyl-pyrrolidine.

13. Supports selon la revendication 11 dans lesquels le polymère greffé est un polymère hydrophile de type polyoxyde d'ethylène PEO aminé.

14. Supports selon la revendication 11 dans lesquels la ou les macromolécule(s) greffée(s) sont un ou des ligands spécifiques de récepteurs cellulaires.

15. Dispositif de préparation en continu de support 2D-MS doués de propriétés particulières, préparés selon un procédé tel que décrit ci-dessus et mettant en oeuvre une fixation covalente d'un polymère, copolymère ou macromolécules biologiques sur un substrat constitué d'un film polymère, ledit dispositif comprenant :
- un système de déroulement/enroulement du film polymère, l'entraînement du film étant effectué à une vitesse choisie au niveau de chacune des étapes : activation, greffage, poinçonnage ;
- un accélérateur d'électrons équipé de manière à activer en continu des bobines de films ;
- un récipient destiné à contenir la solution de monomères, polymères ou macromolécules à greffer, dans lequel le film polymère passe de façon continue à une vitesse déterminée et entraîné par le système d'enroulement/déroulement ;
- un outil de découpe permettant de réaliser le poinçonnage du film lors de son déroulement.

16. Dispositif selon la revendication 15 dans lequel le système de déroulement/enroulement du film permet l'avancement dudit film à une vitesse comprise entre 0,05 et 8 m par minute.

17. Dispositif selon la revendication 15 ou 16 dans lequel l'outil de découpe est constitué d'un bloc outil sur lequel sont enfichées, dans le sens de la longueur du film, des rangées de poinçons circulaires en carbure céramique, et le bloc empreinte correspondant.
